# EUROPEAN PATENT APPLICATION

(11) **EP 0 713 653 A1**
(43) Date of publication of application: **29.05.1996**
(21) Application number: 95308080.1
(22) Date of filing: 10.11.1995
(51) Int. Cl.: A23L 2/52, A23L 1/30, A23L 1/304

(54) **Fortified fruit juice**

(30) Priority: 23.11.1994 GB 9423625
(71) Applicant: SCOTIA HOLDINGS PLC, Guildford Surrey GU1 1BA (GB)
(72) Inventor: Horrobin, David Frederick, Dr., c/o Scotia, Guildford, Surrey GU1 1BA (GB)
(74) Representative: Sturt, Clifford Mark

(57) **Abstract**

A fruit juice enriched or supplemented with GLA and/or DGLA, the fruit juice comprising 1mg to 30g/100ml, preferably 10mg to 15g/100ml and very preferably 100mg to 2g/100ml of GLA and/or DGLA and optionally further comprising other fatty acids such as AA, or SA, or EPA, or DHA in amounts from 1mg to 30g/100ml, preferably 10mg to 15g/100ml and very preferably 100mg to 2g/100ml.

## Description

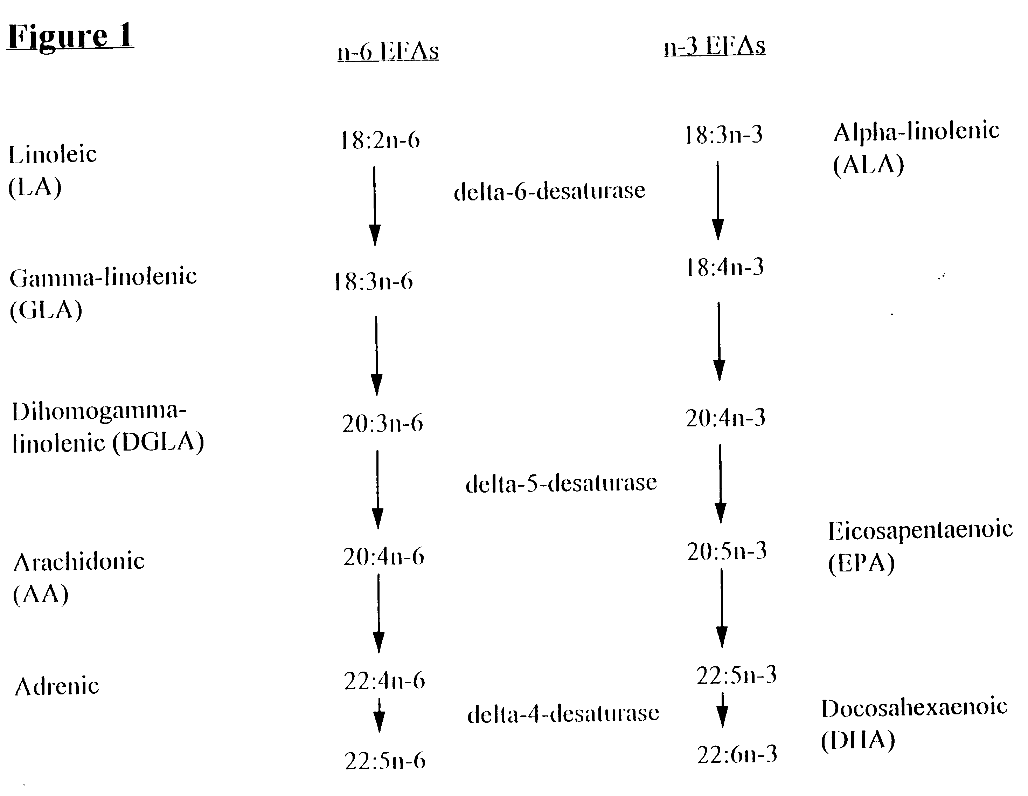

Essential fatty acids (EFAs) are extremely important for human health. There are twelve of these as set out in figure 1. Most diets contain reasonably substantial amounts of the parent fatty acids, linoleic acid (LA), and alphalinolenic acid (ALA). However, in order to perform most of their functions within the body, LA and ALA must first undergo 6-desaturation, by the first, and rate limiting, enzyme delta-6-desaturase and then be converted to the further fatty acids shown in the figure. Among these, dihomogammalinolenic acid (DGLA) and arachidonic acid (AA) of the omega-6 series and eicosapentaenoic acid (EPA) and docosahexaenoic acid (DHA) of the omega-3 series, are particularly important. Gamma-linolenic acid (GLA) and stearidonic acid (SA) are also important in terms of intake to the body because they are beyond the rate-limiting step and can be converted to the other fatty acids relatively easily.

GLA and DGLA have a wide variety of desirable effects and are particularly valuable in the promotion of human health. In humans, 6-desaturase seems particularly slow acting. Only 10-20% of dietary ALA and only 1-3% of dietary LA are converted to their metabolites. This explains why human milk, unlike cows' milk, contains substantial amounts of DGLA, GLA, AA, EPA and DHA. Human babies need these fatty acids to be provided because they cannot make them rapidly enough themselves. Until very recently, artificial infant formulae contained either linoleic acid alone or LA + ALA but did not contain further metabolites. Now infant formulae have been made containing GLA, AA and DHA and several patents have been filed in this field, for example, EP-0140805B and US-4670285. None has involved a final GLA concentration of more than 100mg/100ml.

However, it is not only infants who benefit from the metabolites of the parent fatty acids. The n-6 EFAs, GLA, DGLA and AA, are of value in older children and adults in many situations including skin disorders such as eczema and acne; diabetes and its complications; hypertension and other cardiovascular disorders including coronary and peripheral vascular disease; inflammatory disorders such as rheumatoid and osteo-arthritis, systemic lupus erythematosus, Crohn's disease, ulcerative colitis and many others; respiratory disorders such as asthma; psychiatric disorders such as schizophrenia and alcoholism; gastrointestinal disorders such as peptic ulceration; some cancers; osteoporosis; prostatic disorders; and impaired renal function. Omega-3 fatty acids such as EPA and DHA may also be of value, and many of the above disorders may benefit from co-administration of EFAs from both the omega-6 and omega-3 series.

Much attention has been paid to the omega-3 series but much less attention has been directed to the omega-6 series. It is the inventor's contention that GLA and DGLA are of particular importance in human health.

It is important to be able to present the EFAs in a way which is attractive and pleasant for the consumer. Fruit juices, natural, synthetic, or semi-synthetic are well accepted by most consumers of all ages. They are therefore good vehicles for the administration of fatty acids such as GLA and DGLA and optionally others such as AA, SA, EPA and DHA. The fatty acids may be provided in glyceride, ester, salt, free acid, phospholipid or other appropriate forms, although glycerides, ethyl esters and phospholipids are of particular value because in general they are bland tasting. Any acceptable emulsifying agent or system known to those skilled in the art may be used. However, we have found that while conventional phospholipid and synthetic emulsifiers can be used, galactolipids from plant sources and milk sphingolipids are of particular value in producing stable emulsions. Because the fatty acids are easily oxidised, it is desirable to add an appropriate anti-oxidant. We have found that, while a number of commonly used anti-oxidants are of value, the ascorbate/phosphorylated mono- or di- acyl glyceride system described in earlier patent applications (such as EP-0577305A) is of particular value.

GLA may be obtained as a glyceride or as various fully or partially purified derivatives from many sources including the oils of evening primrose, borage, blackcurrant, and various fungi and algae including Mucor, Rhizopus and Spirulina. DGLA can be synthesised from GLA or isolated from various animal tissues, particularly liver, kidneys, adrenals or gonads. AA can be isolated from the same animal tissues or from egg yolk or obtained from various fungal and algal oils. SA is found in a number of vegetable oils including blackcurrant and blue bur (Lappula Squarrosa) oils and in fungal and algal oils. EPA and DHA are found in marine oils and various algal and fungal oils. All the fatty acids can be chemically synthesised although at present the methods are difficult and expensive.

The invention is particularly directed to the addition of GLA and/or DGLA in therapeutically useful quantities to fruit juices of any type, whether natural or synthetic.

Either fatty acid in any appropriate form may be incorporated into a fruit juice emulsion at levels of, for example, from lmg to 30g/100ml, preferably 10mg to 15g, and very preferably 100mg to 2g/100ml. 100mg/day and above is a therapeutically useful dose, although lower rates of supplementation may be of value in healthy people. Glycerides, phospholipids and esters are the preferred forms because they have relatively bland tastes. However, any other derivatives which can be made palatable and which deliver the GLA or DGLA in an assimilable form are acceptable. It is difficult to prepare emulsions in which the total fatty acid concentration is above about 30g/100ml. Any form of natural or artificial fruit juice, such as orange, apple, grapefruit, peach, pineapple, melon, blackcurrant, kiwi, strawberry, raspberry, guava, pear or juice made from any other fruit may be used. The juice emulsions may be flavoured and/or sweetened in any appropriate way using any appropriate flavours or sweeteners. The emulsions may also be enriched with other nutrients such as calcium, vitamin C, or any other water soluble or fat soluble nutrient substance. The emulsions may also be used as vehicles for natural or synthetic products, with therapeutic potential. The emulsions may be presented in unsterilised, pasteurised or sterilised forms as required. The outer packaging may be anything appropriate for fruit juice.

### Aspects of the Invention

1. Any type of fruit juice enriched or supplemented with 1mg to 30g/100ml, preferably 10mg to 15g/100ml and very preferably 100mg to 2g/100ml of GLA and/or DGLA. Other fatty acids such as AA, or SA, or EPA, or DHA may be optionally added in amounts from 1mg to 30g/100ml, preferably 10mg to 15g/100ml and very preferably 100mg to 2g/100ml.
2. Any type of fruit juice enriched or supplemented with GLA and/or DGLA as in (1), with or without additional calcium and optionally with the addition of other fatty acid(s), particularly EPA, for the enhancement of calcium absorption, of calcium balance and the treatment and prevention of osteoporosis. The calcium may be added in any appropriate form so that 50mg to 2000mg of additional calcium are added to 100ml fruit juice. Appropriate sources of calcium include, but are not limited to, the calcium salt of an essential fatty acid, calcium chloride, calcium gluconate, calcium lactate, calcium carbonate, calcium phosphate, calcium levulinate and calcium glubionate.
3. Any type of fruit juice enriched or supplemented with GLA and/or DGLA as in (1) for the prevention and/or treatment of any of the disorders listed herein.
4. Any type of fruit juice enriched or supplemented with GLA and/or DGLA as in (1) for the treatment and prevention of atopic eczema.
5. Any type of fruit juice enriched or supplemented with GLA and/or DGLA as in (1) optionally with EPA or DHA, for the prevention and treatment of rheumatoid or osteo-arthritis.
6. Any type of fruit juice enriched or supplemented with GLA and/or DGLA as in (1) optionally with EPA, DHA or AA for the treatment of schizophrenia or alcoholism.
7. Any type of fruit juice enriched or supplemented with GLA and/or DGLA as in (1) optionally with AA, EPA, or DHA for the prevention or treatment of hypertension, coronary artery disease or peripheral vascular disease.
8. Any type of fruit juice enriched or supplemented with GLA and/or DGLA as in (1) optionally with other fatty acids or nutrients such as ascorbic acid or ascorbate in amounts of 10mg to 2000mg/100ml for the treatment or prevention of asthma and of diabetes and its complications.
9. Any type of fruit juice enriched or supplemented with GLA and/or DGLA as in (1), optionally with other fatty acids and other nutrients such as zinc in amounts from 1mg to 800mg/100ml for the treatment and prevention of prostatic diseases, including prostatic hypertrophy.
10. Any type of fruit juice enriched with GLA and/or DGLA as in (1) optionally with other fatty acids and other nutrients such as ascorbic acid or ascorbate in amounts from 10mg to 2000mg/100ml, for the supportive nutrition of patients with cancer.

The enriched fruit juices may be prepared by any method known to those skilled in the art. However, one convenient method which is easily applied in practice is to take the GLA or DGLA containing oil, add to 0.1 - 5.0% of an appropriate emulsifier, which could be a phospholipid (such as phospholipid derived from eggs or from plants such as soya beans) or a synthetic emulsifier, but is preferably a plant galactolipid or a milk sphingolipid, and then to add this to the juice in equipment which permits intensive mixing.

### Examples of Formulations

All the formulations may be made using 0.1-5% of galactolipid, sphingolipid or phospholipid added to the fatty acid source as emulsifying agents in addition to the natural emulsifiers of the fruit juice itself.

1. Orange juice, natural or synthetic containing a fatty acid glyceride or phospholipid derived from any of the sources mentioned in the text which provides a level of 100-2000mg of GLA per 100ml.
2. As (1) but with DGLA.
3. As (1) or (2) but with two, three or four other fatty acids selected from AA, SA, EPA or DHA.
4-6. As (1-3) but prepared with additional calcium from any appropriate source but particularly the ones mentioned in the text to provide an extra 50-1000mg/100ml thereof.
7-9 As (1-3) but prepared with additional ascorbic acid or ascorbate to provide an extra 10-2000mg/100ml thereof.
10-12. As (1-3) but prepared using grapefruit juice or apple juice, optionally with added calcium or ascorbic acid as in examples 4-6 and 7-9.

The above examples may be formulated with any appropriate juice, any appropriate flavouring agent, any appropriate colouring, any appropriate stabilising agent, and any appropriate emulsifying agent with the galactolipids or milk sphingolipids at levels of 0.1-5% of the fatty acids by weight being particularly valuable. Phospholipids or sphingolipids from any appropriate source such as soya beans, milk, eggs or other source may also be used.

### Examples of Galactolipid Emulsifiers

With regard to the galactolipids, two types of classes of lipids based on galactose are very common, that is mono-galactosyldiacylglycerol and digalactosyldiacylglycerol. Commonly used nomenclature and abbreviations are monogalactosyldiglyceride, MGDG, and digalactosyldiglyceride, DGDG. The general structure of said compounds are outlined below. 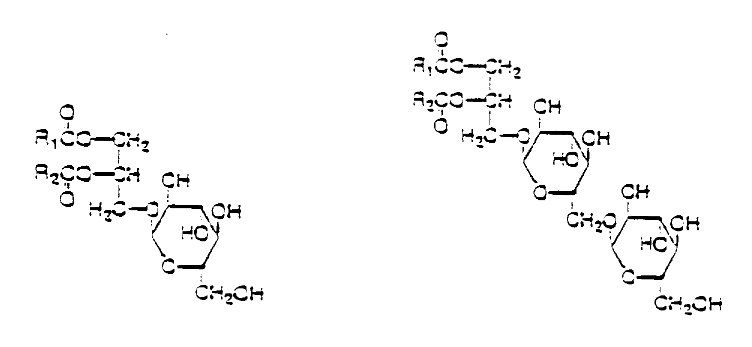 wherein R₁, R₂ = fatty acid residues
MGDG DGDG

To some extent one of the two fatty acid residues may be absent in the two lipids, which results in the corresponding monoacyl compounds, i.e. digalactosylmonoglyceride, DGMG, and monogalactosylmonoglyceride, MGMG. Higher homologues with additional galactose units may also occur in small amounts.

### Examples of Galactolipid Extraction

The glycosylglyceride material can be extracted from almost any kind of glycolipid-containing plant material. Preferred plant materials are seeds and kernels from grains and cereals, for instance, wheat, rye, oats, corn, rice, millet and sesame.

The following relates to a procedure for producing a glycolipid material from oat kernels.

200kg of oat kernels (Kungsörnen, Sweden) were grounded and extracted with 1000 l of 95% ethanol at 70°C for 3 h in an extraction tank under stirring. The slurry was centrifuged while still warm and separated from solid particles. The liquid fraction was evaporated at maximum 60°C which yielded about 10kg of extract in the form of a light brown oil.

The carbohydrates were removed in the following way:
10kg of the extract was carefully mixed with 10 l of hexane (hexane polymerisation grade, distillation range 65-70°C, Shell) and 50 l of 70% aqueous ethanol. The mixture separated into two phases over night. The lower, more polar phase containing the carbohydrates was discarded. The lipids are contained in the upper phase.

The upper phase, amounting to about 20 l, was applied to a stainless steel column of 30cm x 20cm (ID x h) having a volume of about 14 l and containing 6.25kg of silica gel (Matrex Silica Si, particle size 20-45 µm, pore diameter 60 Å, from Amicon Corporation, Danvers). The column was heated to 50°C and then washed with 30 l of a mixture of hexane:isopropanol, 90:10, in order to remove all triglycerides.

The glycolipid material was then eluted from the column with 20 l of a mixture of hexane:isopropanol, 60:40, giving a glycolipid fraction. Evaporation of the glycolipid fraction gave about 700g of a glycolipid material.

Analysis of the glycolipid material by HPLC indicated that the glycolipid material contains >70 area % DGDG. Two of the peaks, DGDG1 and DGDG2, respectively, represent two major classes of digalactosyldiglycerides having different acyl group structures. Some of the additional peaks have been identified as phospholipids, that is PE, phosphatidylethanolamine, PA, phosphatidic acid, PI, phosphatidylinositol and PC, phosphatidylcholine. The total amount of phospholipids is below 10 area %.

## Claims

1. A fruit juice enriched or supplemented with GLA and/or DGLA, the fruit juice comprising 1mg to 30g/100ml, preferably 10mg to 15g/100ml and very preferably 100mg to 2g/100ml of GLA and/or DGLA and optionally further comprising other fatty acids such as AA, or SA, or EPA, or DHA in amounts from 1mg to 30g/100ml, preferably 10mg to 15g/100ml and very preferably 100mg to 2g/100ml.

2. A fruit juice according to claim 1 further comprising additional calcium in any appropriate assimilable form, providing 50mg to 2000mg of additional calcium per 100ml and optionally further comprising other fatty acid(s), particularly EPA.

3. A fruit juice according to claim 1 or 2 for the enhancement of calcium absorption, of calcium balance and the treatment and prevention of osteoporosis.

4. A fruit juice according to claim 1 for the prevention and/or treatment of any of the disorders listed herein.

5. A fruit juice according to claim 1 for the treatment and prevention of atopic eczema.

6. A fruit juice according to claim 1 optionally with EPA or DHA, for the prevention and treatment of rheumatoid or osteo-arthritis.

7. A fruit juice according to claim 1 optionally with EPA, DHA or AA for the treatment of schizophrenia or alcoholism.

8. A fruit juice according to claim 1 optionally with AA, EPA, or DHA for the prevention or treatment of hypertension, coronary artery disease or peripheral vascular disease.

9. A fruit juice according to claim 1 optionally with other fatty acids or nutrients such as ascorbic acid or ascorbate in amounts of from 10mg to 2000mg/100ml for the treatment or prevention of asthma and of diabetes and its complications.

10. A fruit juice according to claim 1 optionally with other fatty acids and other nutrients such as zinc in amounts from 1mg to 800mg/100ml for the treatment and prevention of prostatic diseases, including prostatic hypertrophy.

11. A fruit juice according to claim 1 optionally with other fatty acids and other nutrients such as ascorbic acid or ascorbate in amounts from 10mg to 2000mg/100ml, for the supportive nutrition of patients with cancer.

12. A fruit juice according to any preceding claim wherein the fruit juice is prepared using a natural or synthetic juice.

13. A fruit juice according to any preceding claim wherein the fruit juice is prepared using orange, grapefruit, apple, peach, pineapple, melon, blackcurrant, kiwi, strawberry, raspberry, guava or pear juice.

14. A fruit juice according to any preceding claim further comprising an appropriate emulsifier such as phospholipid or a synthetic emulsifier, preferably a plant galactolipid or a milk sphingolipid.
